**Europäisches Patentamt**

**European Patent Office**

**Office européen des brevets**

(11) Veröffentlichungsnummer: **0 271 034
B1**

(12) **EUROPÄISCHE PATENTSCHRIFT**

(45) Veröffentlichungstag der Patentschrift:
**21.06.89**

(51) Int. Cl.⁴: **C07C 2/20**, C10G 71/04,
C10M 105/04

(21) Anmeldenummer: **87118010.5**

(22) Anmeldetag: **05.12.87**

(54) Verfahren zur Herstellung von Decenoligomeren und deren Verwendung als Schmieröle.

(30) Priorität: **12.12.86 DE 3642456**

(43) Veröffentlichungstag der Anmeldung:
**15.06.88 Patentblatt 88/24**

(45) Bekanntmachung des Hinweises auf die Patenterteilung:
**21.06.89 Patentblatt 89/25**

(84) Benannte Vertragsstaaten:
**AT BE DE FR GB IT NL**

(56) Entgegenhaltungen:
**WO-A-82/04040
US-A- 3 382 291**

(73) Patentinhaber: **BASF Aktiengesellschaft,
Carl-Bosch-Strasse 38, D-6700 Ludwigshafen(DE)**

(72) Erfinder: **Bronstert, Klaus, Dr., Gartenstrasse 26,
D-6719 Carlsberg(DE)**
Erfinder: **Mach, Helmut, Dr., Dantestrasse 5,
D-6900 Heidelberg(DE)**
Erfinder: **Rath, Hans Peter, Dr., Friedhofstrasse 7,
D-6718 Gruenstadt(DE)**
Erfinder: **Walter, Hans-Michael, Dr., Im Haagweg 6,
D-6701 Ruppertsberg(DE)**

**Beschreibung**

Verfahren zur Herstellung von Decenoligomeren und deren Verwendung als Schmieröle

Die Erfindung betrifft ein Verfahren zur Herstellung von Decenoligomeren durch Oligomerisierung von Decen-1 in Gegenwart von Bortrifluorid als Katalysator und Wasser als Cokatalysator bei Temperaturen von 0 bis +50°C.

Bei derartigen Verfahren werden Decenoligomere erhalten, die für synthetische Schmieröle Verwendung finden.

Die bekannten Katalysatoren für die Herstellung der Decenoligomeren besitzen jedoch gewisse Nachteile, wie z.B. niedrige Umsätze des Decen-1, Oligomerisierung in mehreren Reaktionsstufen und aufwendiges Abtrennen des Decen-1 aus dem Reaktionsgemisch.

In der US-Patentschrift 3,382,291 wird ein Verfahren zur Herstellung von Decenoligomeren unter Verwendung von Bortrifluorid als Katalysator und z.B. Wasser als Cokatalysator beschrieben. Das Decen wird dabei mit $BF_3$ gesättigt und in einer Reaktionszone mit einem 1:1 molaren Komplex von $BF_3$ und Cokatalysator gemischt. Ein ähnliches Verfahren ist in der US-Patentschrift 3,769,363 beschrieben, wobei unter einem $BF_3$-Druck von 50 bis 100 mm Hg gearbeitet wird. In beiden Verfahren ist der Decenumsatz unvollständig und eine Rückführung von nicht umgesetzten Decen aus dem Produktstrom in die Reaktionszone ist notwendig.

Aus der britischen Patentschrift 1 497 524 ist ebenfalls die Decenoligomerisierung mit $BF_3$ bekannt, wobei als Cokatalysator organische Verbindungen wie Alkohol, Ether, usw. genannt werden. Der $BF_3$-Druck beträgt 0,35 bis 35 kg/cm². In einem ersten Reaktor wird eine partielle Oligomerisierung durchgeführt. Die weitere Umsetzung erfolgt in einem oder mehreren Folgereaktoren.

In den genannten Verfahren wird entweder mit Wasser als Cokatalysator oder bei hohem $BF_3$-Druck oligomerisiert. In allen Fällen ist der Umsatz unvollständig oder es muß in mehreren Reaktoren oligomerisiert werden.

Aufgabe der vorliegenden Erfindung war es, die oben geschilderten Nachteile zu vermeiden und ein technisch einfaches Verfahren zur möglichst vollständigen Oligomerisierung von Decen-1 aufzufinden.

Diese Aufgaben wurden durch Verfahren gemäß Patentansprüchen 1 bis 2 gelöst.

Die üblichen Verfahren zur Herstellung von Decenoligomeren mit Hilfe von Katalysator-/Cokatalysator-Kombinationen der eingangs beschriebenen Art sind, z.B. aus der oben zitierten Literatur, so bekannt, daß sich für den Fachmann eine weitere Erläuterung erübrigt. Als besonders geeignetes Bortrifluorid wird üblicherweise bevorzugt ein $BF_3$ mit einem Reinheitsgrad von 99 % oder besser verwendet. Unter Oligomeren werden bekanntlich Polymerisationsprodukte verstanden, die aus wenigen Monomeren bestehen, d.h. einen niedrigen Polymerisationsgrad aufweisen. Bevorzugt weisen die Decenoligomeren eine kinematische Viskosität bei 100°C von 3 bis 200 mm²/s auf (gemessen nach DIN 51 562).

Es wurde nun gefunden, daß durch Kombination von Wasser als Cokatalysator und hohem $BF_3$-Partialdruck eine vollständige Oligomerisierung von Decen-1 in einer einzigen Reaktionsstufe erreicht werden kann, so daß sich eine aufwendige Abtrennung des Decen-1 aus dem Reaktionsgemisch erübrigt.

Gemäß der Erfindung umfaßt das Verfahren zur Durchführung der Oligomerisierung von Decen-1, in dem ein vollständiger Decenumsatz erreicht wird, das Vorlegen der Decen/Wasser-Mischung in einem Rührreaktor bei Temperaturen von 0 bis +25°C, vorzugsweise +5 bis +15°C, und Aufpressen von Bortrifluorid bis zu einem Druck von 2 bis 10 bar, vorzugsweise 3 bis 6 bar. Durch Absorption und Reaktion verbrauchtes $BF_3$ kann nachgeführt werden, so daß der $BF_3$-Druck im Verlauf der Oligomerisierung weitgehend konstant bleibt. Das Wasser als Cokatalysator wird in einer Menge von 0,5 bis 5,0 Gew.%, vorzugsweise 0,8 bis 2,0 Gew.%, bezogen auf Decen-1, eingesetzt. Durch entsprechende Außenkühlung wird die Reaktionstemperatur zwischen 0 und +50°C gehalten. Es ist eine Reaktionszeit von mindestens 20 Minuten einzuhalten. Im weiteren Verlauf bis zu 2 Stunden Reaktionszeit verschiebt sich das Verhältnis der Anteile der verschiedenen Oligomeren, so daß über die Reaktionszeit die Produktzusammensetzung kontrolliert werden kann. Eine längere Verweilzeit bringt praktisch keine Veränderung mehr und ist daher nicht sinnvoll.

Die Aufarbeitung des Produkts erfolgt in der an sich bekannten Art und Weise durch Waschen des Reaktionsgemischs mit verdünnter Natronlauge und Wasser zur Entfernung des Katalysators.

Der Decenumsatz kann beispielsweise durch fraktionierte Destillation oder durch Gelpermeationschromatographie (GPC) bestimmt werden. Ein geeignetes GPC-System besteht aus einer 25 cm ®Lichrogelsäule PS4 (Merck, Darmstadt), auf der das Reaktionsgemisch mit Tetrahydrofuran als Laufmittel mit einer Flußrate von einem ml/min getrennt wird. Die Detektion erfolgt z.B. mit einem Brechungsindexdetektor.

Bei synthetischen Schmierölen ist die Verwendung eines vollkommen gesättigten Materials wünschenswert. Dazu kann das Reaktionsgemisch hydriert werden, um irgendwelche vorliegende Nichtsättigung zu vermeiden. Verfahren zur Herstellung von synthetischen Schmiermitteln auf Basis von Decenoligomeren sind in der US-Patentschrift 3,149,178 beschrieben.

Das folgende Beispiel dient lediglich zur Erläuterung der Erfindung, ohne sie zu begrenzen.

Beispiel

In einem 250 ml-Autoklaven wurden 100 g Decen-1 und 0,15 g Wasser (8,33 mmol) eingefüllt und auf 15°C thermostatisiert. Es wurde kurz evakuiert und anschließend $BF_3$ aufgepreßt (5 bar). Innerhalb von 10 min fiel der Druck um 1 bar und die Temperatur stieg auf +43°C an. Der $BF_3$-Druck wurde wieder auf 5 bar eingestellt. Das Nachpressen von 4 auf 5 bar mußte noch 2 x wiederholt werden.

Es wurden Proben nach 20 min und 1 Stunde genommen und ebenso wie das Endprodukt nach 2 Stunden durch GPC untersucht. Demnach ergibt sich folgende Zusammensetzung in Flächen-% (Fl.-%):

| Reaktionszeit (min) | Decen-1 (Fl.-%) | Dimer + Trimer (Fl.-%) | Tetramer u. höhere Oligomere (Fl.-%) |
|---|---|---|---|
| 20 | 2,71 | 56,51 | 40,78 |
| 60 | – | 48,28 | 51,59 |
| 120 | – | 38,48 | 61,52 |

Die Aufarbeitung der GPC-Proben und der Hauptmenge erfolgte durch mehrmaliges Waschen a) mit 5 gew.%iger NaOH und b) mit Wasser. Nach dem Trocknen über $MgSO_4$ wurde die Hauptmenge auch destillativ auf Restmonomer geprüft. Bis zu einer Badtemperatur von 225°C erfolgte kein Übergang, der Monomerumsatz war also vollständig gewesen. Nach GPC enthält das Endprodukt ebenfalls kein Decen-1. Die kinematische Viskosität betrug 6,4 cSt bei 100°C, bestimmt nach DIN 51 562.

Vergleichsbeispiel

In einem 250 ml-Autoklaven wurden 100 g Decen-1 und 1,32 g Decanol (8,33 mmol) eingefüllt und auf 15°C thermostatisiert. Es wurde kurz evakuiert und anschließend $BF_3$ aufgepreßt (100 mm Hg). Es wurde kein Temperaturanstieg beobachtet. Der $BF_3$-Druck wurde während der gesamten Reaktionszeit gehalten.

Es wurden Proben nach 20 min und 1 Stunde genommen und ebenso wie das Endprodukt durch GPC untersucht. Es wurde die folgende Zusammensetzung gefunden (Flächen-%).

| Reaktionszeit (min) | Decen-1 (Fl.-%) | Oligomere (Fl.-%) |
|---|---|---|
| 20 | 42,37 | 57,63 |
| 60 | 8,13 | 91,87 |
| 120 | 2,45 | 97,55 |

Die Aufarbeitung der GPC-Proben und der Hauptmenge erfolgte durch mehrmaliges Waschen mit a) 5 %iger NaOH und b) mit Wasser und Trocknen mit $MgSO_4$.

**Patentansprüche**

1. Verfahren zur Herstellung von Decenoligomeren durch Oligomerisieren von Decen-1 in Gegenwart von Bortrifluorid als Katalysator und Wasser als Cokatalysator bei Temperaturen von 0 bis +50°C, dadurch gekennzeichnet, daß in einem Rührreaktor eine Decen-/Wasser-Mischung vorgelegt und das Bortrifluorid bis zu einem $BF_3$-Partialdruck von 2 bis 10 bar über einen Zeitraum von 20 bis 120 Minuten aufgepreßt wird.

2. Verfahren nach Anspruch 1, dadurch gekennzeichnet, daß der $BF_3$-Partialdruck 3 bis 6 bar beträgt.

3. Verwendung der Decenoligomeren nach Anspruch 1, gegebenenfalls in hydrierter Form, als Grundflüssigkeit für synthetische Schmieröle.

**Claims**

1. A process for preparing a decene oligomer by oligomerizing decene-1 in the presence of boron trifluoride as catalyst and water as cocatalyst at from 0 to +50°C, which comprises introducing initially a decene-1/ water mixture into a stirred reactor and injecting the boron trifluoride on top up to a $BF_3$ partial pressure from 2 to 10 bar in the course of a period from 20 to 120 minutes.

2. A process as claimed in claim 1, wherein the $BF_3$ partial pressure ranges from 3 to 6 bar.

3. Use of a decene oligomer as claimed in claim 1, with or without hydrogenation, as a base for synthetic lubricating oils.

**Revendications**

1. Procédé de préparation d'oligomères de décène par oligomérisation de décène-1 en présence de trifluorure de bore servant de catalyseur et d'eau servant de co-catalyseur, à des températures de 0 à +50°C, caractérisé en ce qu'on charge préalablement, dans un réacteur comportant un agitateur, un mélange de décène-1 et d'eau, et en ce qu'on comprime le trifluorure de bore jusqu'à une pression partielle de $BF_3$ de 2 à 10 bars au cours d'une période de temps de 20 à 120 mn.

2. Procédé selon la revendication 1, caractérisé en ce que la pression partielle de $BF_3$ est de 3 à 6 bars.

3. Utilisation d'oligomères de décène selon la revendication 1, le cas échéant sous forme hydrogénée, comme liquide de base pour des huiles lubrifiantes synthétiques.